Europäisches Patentamt

European Patent Office

Office européen des brevets

(11) Publication number: **0 322 240 B1**

# EUROPEAN PATENT SPECIFICATION

(45) Date of publication of patent specification: **01.03.95**

(51) Int. Cl.[6]: **C12N 15/00**, C12P 21/00

(21) Application number: **88312222.8**

(22) Date of filing: **22.12.88**

(54) **Chimeric immunocompromised mammals and their use.**

(30) Priority: **23.12.87 US 137173**

(43) Date of publication of application:
**28.06.89 Bulletin 89/26**

(45) Publication of the grant of the patent:
**01.03.95 Bulletin 95/09**

(84) Designated Contracting States:
**AT BE CH DE ES FR GB GR IT LI LU NL SE**

(56) References cited:

**SCIENCE, Vol. 241 (1988) pp. 1632-1639 J.M.
McCUNE et al.: "The SCID-hu Mouse:murine
model for the analysis of human hematolym-
phoid differentiation and function"**

**SCIENCE, Vol. 241 (1988) pp. 1581-1583 G.D.
YANCOPOULOS et al.: "Reconstruction of an
immune system"**

**J. OF IMMUNOLOGY, Vol. 136 (12),(1986) pp.
4438-4443. FULOP GM et al.: "Fullreconstitu-
tion of the immune deficiency in Scid Mice
with normal stem cells requires low-dose
irradiation of the recipients"**

(73) Proprietor: **THE BOARD OF TRUSTEES OF THE
LELAND STANFORD JUNIOR UNIVERSITY
Encina 105
Stanford University
Stanford
California 94305 (US)**

(72) Inventor: **McCune,Joseph,McCrary
51Clipper
San Francisco,California 94414 (US)**

(74) Representative: **Harrison, David Christopher
et al
MEWBURN ELLIS
York House
23 Kingsway
London WC2B 6HP (GB)**

**Description**

The field is the growth of normal xenogeneic cells in immunocompromised non-human animals. Particularly, xenogeneic hematopoietic cells and organs associated with the hematopoietic system are produced.

An important area of biological interest has been the production and use of transgenic animals, where embryonic cells are modified, resulting in mosaicism, where neonates having different phenotypes from their parents are obtained, where xenogeneic products are produced in a mammalian fluid, e.g. milk, where the differences in the differentiated host may be used in the understanding of physiological processes, disease susceptibility and transmission, production of products, and the like.

In many ways, the human host is unique among all of the mammals. Homicide is uniquely applied to the killing of man and the laws are much more stringent in relation to activities affecting man as compared to other species.

In studying drugs and other therapeutic treatments, animals are first tested to determine whether the drug or treatment may be safe for humans. Only rarely can humans be used for scientific investigation, so that in many instances the treatment must first be tested in lower animals, before it may be used with man. Therefore, developing human products and evaluating diseases which are peculiar to man are very difficult, since frequently it is difficult to obtain a human product outside of a human host or to develop an animal model which can be safely extrapolated to human experience. In many instances, the experiences associated with animals, e.g. mice, rabbits, rats and even primates, have proven to be poor predictors of experience with humans.

There is, therefore, substantial interest in being able to develop systems which would allow for the production of human products, including human cells or tissue. The ability to produce these products in commercially significant amounts would have an extraordinary stimulating effect on the ability to study human processes, both natural and diseased, as well as the use of human products in the diagnosis and treatment of physiological processes.

Relevant Literature

References concerned with immunoincompetent hosts, particularly CID, or SCID hosts include McGuire et al., Clin. Immunol. and Immunopath. (1975) 3:555-566; Perryman and Torbeck, J. Am. Vet. Med. Assoc. - (1980) 176:1250-1251; Schultz and Sidman, Genetically-determined Murine Models of Immunodeficiency, The Jackson Laboratory, Bar Harbor, ME; Bosma et al., Nature (1983) 301:527-530; Custer et al., Amer. J. Path. (1985) 120:464-477; Dorshkind et al., J. of Immunol. (1985) 134:3798-3801; Kerghtley et al., Lancet, November 1, 1975, 850-853; Touraine, Immunological Rev. (1983) 71:103-121; and Fulop and Phillyes, J. of Immunology (1986) 136:4438-4443.

References concerned with xenogeneic cells growing within live hosts include Krueger et al., J. Inv. Dermatol. (1975) 64:307-312; Krueger et al., Proc. Natl. Acad. Sci USA (1983) 80:1650-1654; Krueger and Shelby, J. Inv. Dermatol. (1981) 76:506-510; Ware et al. J. Immunol. Meth. (1985) 85:353-361; Ford et al., Nature (1956) 177:452-454; Dovlsen et al., Nature (1974) 248:247-249; Mannhardt et al., Thymus (1982) 4:209-220; Schulte-Wisserman et al., Scand. J. Immunol. (1978) 8:387-396. Please specifically note, McCune et al., Science (1988) 241:1632-1639 and the comment therein; Yancopoulos and Alt, Ibid (1988) 241:1581-1583, and references cited therein.

SUMMARY OF THE INVENTION

Methods, compositions and organisms are provided for the propagation of functioning normal cells and organs in a live xenogeneic non-human animal host. Normal donor mammalian cells are introduced into an immunocompromised xenogeneic non-human animal host particularly to complement a defect, and grown in the chimeric host, whereby the donor cells function in substantially normal manner in the chimeric host, differentiate proliferate, produce valuable products native to the donor host, different types of cells interact analogous to their actions in the donor source, and may provide a source of cells and products, particularly for investigation or use with the donor mammalian source.

DESCRIPTION OF THE SPECIFIC EMBODIMENTS

Methods are provided for allowing cells, particularly undifferentiated ("stem") cells, to differentiate and proliferate in a xenogeneic non-human animal, normally mammalian host, where the progeny cells are capable of functioning in the host. The cells grow and produce products native to the cells, including progeny cells, which may find use in research, production of antibodies, production of physiologically active products, as transplants, and in numerous other applications. The method involves introduction of the cells in an appropriate site or environment in the animal host to provide a chimeric host. Particularly, more than one type of cell may be introduced into the xenogeneic host, where the different cells may interact providing for maturation, differentiation or expression of cell function.

To the extent that the subject invention is primarily concerned with xenogeneic hosts having a sophisticated immune system, the xenogeneic host will be a non-human mammalian host. To that extent in the following description, the discussion will be directed to mammalian hosts. However, it should be understood that in some instances animal hosts other than mammalian hosts may find use. Such other hosts include birds and fish, animals with less sophisticated immune systems.

The subject method is applicable to introducing mammalian xenogeneic cells, tissue and/or organs ("transplant") which may complement a defect in an immunocompromised host. Desirably, the transplant comprises stem cells. The immunocompromised host may have a xenogeneic immune system which is syn- or allogeneic to the transplant.

Various systems involving stem cells may be employed in the animal host. The subject immunocompromised host may serve as a vessel for a variety of xenogeneic cells and physiologic systems, providing for an environment in which the cells and systems may differentiate and function.

In many instances, it will be desirable to introduce combinations of cells, where the different cells or organs may interact. For example, hematopoietic stem cells may be introduced into the host in conjunction with embryonic yolk sac, fetal liver, thymus, spleen, or lymph node tissue, fetal or adult bone marrow tissue, pancreatic tissue, appendix tissue, tonsil tissue, and the like. Other stem cell systems may be employed. In other than the hematopoietic system, combinations may include stem cells to the central or peripheral nervous system, such as neuronal or matrix cells of the fetal central nervous system, spinal cord neurons and the spinal cord, organs of the endocrine system, such as $\beta$-cells and other cells of the islets and pancreas, the adrenal gland, the thyroid gland, and the hypothalamo-pituitary axis. These stem cells would be introduced in combination with the appropriate organs in which each of them is induced to differentiate and to become functional.

Exemplary of complementing a defect is the use of hematopoietic cells and organ tissue, where combinations of stem cells (precursor cells for the various human hematopoietic lineages) and cells providing a processing organ, such as the thymus, in conjunction with bone marrow, spleen, and/or lymph node, may be introduced into the mammalian xenogeneic immunocompromised host to produce the various differentiated cells, such as monocytes, macrophages, B-cells, T-cells, neutrophils, erythrocytes, eosinophils, platelets, and the like, and provide these cells in the peripheral blood circulation. The differentiated cells will populate the peripheral blood organs such as the spleen, pancreas, lymph nodes, tonsils, etc., particularly when such organs are syn- or allogeneic. Thus, hematopoietic stem cells in conjunction with the appropriate processing organ tissues can provide for maturation (differentiation) of the stem cells to the various intermediate and mature hematopoietic lineages.

In some cases the implanted xenogeneic tissues/organs may represent targets for disease agents, e.g., joint synovia and/or thyroid tissue to study autoimmune disease, or liver or cardiac tissue to study human hepatotropic and cardiotrophic viruses, respectively.

Immunocompromised non-human mammalian hosts having the desired immune incapacity exist or can be created. For example, hosts with severe combined immunodeficiency, known as scid/scid hosts, are available. Rodentia, particularly mice, and equine, particularly horses, are presently available as scid/scid hosts (hereafter referred to as SCID hosts). The SCID hosts lack functioning B-cells and/or T-cells. In the SCID mouse, the genetic defect appears to be a non-functioning recombinase, for the germline DNA is not rearranged to produce functioning surface immunoglobulin and T-cell receptors. The immunocompromised hosts may also be a result of a non-functioning thymus, irradiation, so as to destroy stem cells, treatment with stem cell specific cytotoxic agents, cytotoxic agents specific for rapidly dividing cells, anti-asialogycoprotein GM-l, or the like. At a minimum, usually an immunocompromised xenogeneic host will lack functioning B- and T-cells, particularly as a genetic defect in B- and/or T-cell germline rearrangements. Therefore in many instances the immunocompromised host may have functioning organs associated with the immune systems such as thymus, spleen, lymph node, etc.

The immunocompromised host may be further modified by breeding between an immunocompromised host and a host having another phenotype of interest. For example, the C.B17 scid/scid strain might be backcrossed with suitable murine strains having low or no NK activity. In this way, a mouse strain lacking T-, B-, and NK cells might be produced. Such a host would predictably better accept and maintain xenogeneic tissue and cells.

Alternatively, a host may be mated to introduce a genetic defect into a host, to allow for studies of xenogeneic tissue in a negative background or the effect of xenogeneic tissue on the background defect. For example, a diabetic trait, hematopoietic defects, e.g. β-thalassemia, sickle cell, etc., or the like as described previously may be backcrossed into the host.

Defects may be naturally present or induced. For example, to induce diabetes, immunotoxins may be employed for Langerhans cells. By destroying the mature Langerhans cells, xenogeneic Langerhans cells may be introduced and compounds and conditions studied as to their effect on the xenogeneic Langerhans cells. Parkinsonianism may be created by treatment with MMTA, substantially destroying host dopaminergic cells. The pancreas may be surgically removed and xenogeneic pancreatic tissue introduced. Alternatively, chemical agents may be used to selectively destroy the β-cells of the host's pancreatic islets. The host may be irradiated and xenogeneic bone marrow introduced.

Thus, a wide variety of cells, tissue and organs may be studied in vivo, as well as the effect of various conditions and physiologically active compounds on their viability and functioning. Where the transplanted immune system and transplanted other cells, tissue and organs are at least allogeneic in similarity, the situation is more analogous to the natural situation for the transplanted cells, tissues and organs.

Xenogeneic organs may be introduced, particularly as tissue which may produce human products under the physiologic conditions of the immunocompromised mammalian host. Of particular interest would be tissue which is genetically predisposed to a disease pathway or where such response may be induced, such as Type 1 diabetes; targets of human immune attack, such as synovial tissue; or targets subject to tropic, or microorganism attack, such as the liver with hepatitis A, B or non-A, non-B, or malaria; and the like.

The xenogeneic host will be an immunocompromised mammal, other than human, where, for the most part, the donor cells will be human cells. Although cells from sources other than members of the same family of the xenogeneic host may also find use. The immunocompromised host may be immunocompromised in a variety of ways, where the result will be the substantial lack of functional T- and B-cells. The xenogeneic hosts may have defects at various levels resulting in an immunocompromised host. The defect may result in loss of functional antibody expressing or independent lymphocytes, such as natural killer (NK) cells, lymphokine activated killer (LAK) cells, antibody dependent cytotoxic (ADCC) cells, tumor infiltrating lymphocytes (TIL), macrophages, etc.

Any non-human mammalian host may be employed other than the presently available mice and horses (equine), which hosts may include members of the ovine, bovine, caprine, lagomorpha, primate (other than human), porcine, canine, feline, etc., or other warm-blooded vertebrates, such as birds. Of particular interest are laboratory animals, such as mice, rats, guinea pigs, e.g. capybara, and rabbits, as well as domestic animals, such as primates other than humans, cows, sheep, pigs, or the like. The xenogeneic host will usually be of a different family from the host source of cells.

Depending upon whether the cells to be introduced are to provide a solid organ, various sites may be selected for introduction of the cells. These sites will vary depending upon whether the tissue to be introduced will result in a solid organ or provide for dispersed cells. Sites for introduction may include under the spleen capsule, abdominal wall, muscle, under the renal capsule, the peritoneum, the peritoneal lining, brain, subcutaneous, vascular system, spleen, spinal cord, blood, liver, membranous sacs or capsules of various tissue, the retroperitoneal space, skin, reproductive organs, etc. Usually, progenitor tissue will be introduced which will grow into a functioning organ. Introduction of the tissue may be achieved by injection, implantation, or joining blood vessels (and other vessels if necessary) of the donor and host, using intravenous catheters, trocars and/or surgical incision, or the like. The tissue or cells of interest will generally be normal tissue or cells (as distinguished from neoplastic or defective tissue or cells) and may be associated with the hematopoietic system, the central nervous system, the autonomic nervous system, brain, liver, bone marrow, bone, digestive system, the reproductive system, bladder, gall bladder, joints, pancreas, retina, nerve, spleen, adrenal gland, etc. Of particular interest, as to organs or parts associated with the hematopoietic system, are lymph nodes, liver, thymus, spleen, lymphatic vessels, veins, arteries, valves, capillaries, bone marrow, skin, appendix, tonsils, etc. Other organs which may be employed include tissue from the brain, such as cerebrum, cerebellum, medulla oblongata, cortex, pons, corpus callosum, cerebral peduncle, hippocampus, thalamus, basal ganglia, etc., as well as portions thereof. Other tissue may include placental tissue, synovial tissue, vascular tissue, esophageal tissue, membrane tissue, smooth muscle tissue, muscle tissue, bone tissue, cardiac tissue, cartilage, mucosal membranes, etc.

In some instances, defective tissue or cells may be grown to investigate the genetic disease, expand the source of defective DNA, investigate the effect of drugs and treatment regimens, and the like. Tissue of interest may include β-thalassemic cells, cells associated with Huntington's disease or Duchenne's syndrome, cells with fetal anomalies including trisomy 21, cells associated with diabetic pancreas, cells from patients with Alzheimer's disease or myasthenia gravis, and other disorders of the central nervous system, etc.

The source of the tissue may vary from embryo yolk sac to fetal tissue, having a gestational age of at least about 4 weeks, more usually at least about 6 weeks, ranging up to adult tissue, depending upon the nature of the tissue or organ. Preferably, the tissue will be from a child of less than about 3 years, preferably less than about 1 year and at or younger than neonate, more preferably being fetal tissue of from about 7 to 24 weeks.

For different organs differently aged tissue may be preferred. For fetal tissue, human lymph node is desirably equal to or greater than about 15 gestational weeks (g.w.),preferably 16-20 g.w.; for human thymus, from about 9 to 24 g.w., preferably less than about 20 g.w.; bone marrow tissue, from about 16 to 24 g.w.; and for human fetal liver, from about 10 to 24 g.w., preferably from about 13 to 22 g.w.

The tissue may be fresh tissue, obtained within about 48 hrs of death, or freshly frozen tissue, tissue frozen within about 12 hrs of death and maintained at below about -10°C, usually at about liquid nitrogen temperature (-70°C) indefinitely. The tissue may be from an organ implanted in the chimeric host, where the tissue may be removed from 2 to 4 weeks after implantation, or longer. In this manner, the tissue originally obtained from the host source may be greatly expanded, substantially increasing the total number of chimeric hosts which may be obtained. The tissue obtained from the chimeric host may be treated analogously to the tissue obtained from the original tissue source host. The tissue may be provided as individual cells freed of attached stromal elements, as a dispersion, or as small tissue slices, generally of from about 0.5 mm to 4 mm, more usually from about 1 mm to 2 mm, generally of a thickness in the range of about 1 to 2 mm, so that the sections can easily fit into a trocar used for implantation, usually conveniently of about 15- to 20-gauge. Normally, the cells will not have been subject to culture in vitro for any extended period of time, e.g., three days or greater; for special purposes, however, such pre-implantation culture in vitro may prove desirable. In some cases whole organ grafts may be transplanted by anastomosing donor and host blood vessels, lymphatic vessels, and other vessels such as ureters etc.

As appropriate, dispersed cells may be employed, where the relevant organs are teased apart to yield viable cells in suspension. Desirably, the suspension cells may be enriched for the particular cells of interest. For example, with fetal liver cells, the suspension cells may be enriched for hematopoietic precursors by ficoll-hypaque density gradient centrifugation. Cells may also be enriched by other techniques, such as fluorescence-activated cell sorting, panning, magnetic-bead separation, elutriation within a centrifugal field, or rosetting.

In some instances, it may be desirable to enrich cells by killing or removing other cells. This may be achieved by employing monoclonal antibodies specific for the undesired cells in the presence of complement or linked to a cytotoxic agent, such as a toxin, e.g. ricin, abrin, diphtheria toxin, or a radiolabel, e.g. $^{131}$I, or the like. Immunoaffinity columns may be employed, which allow for specific separation of either the desired or undesired cells, depending upon the nature of the mixture.

The recipient mammalian host may be subject to a variety of immune defects. Of particular interest is the defect resulting in non-functional T- and B-cells. Such dysfunction may be achieved with SCID (severe combined immunodeficiency) animals. Current scid/scid species include mice and horses. In the future, e.g., using transgenic cell-depletion techniques, scid animals of other species may be developed. Other malfunctions may include non-functional stem cells, lack of surface membrane proteins associated with T-cell and B-cell function, incompetent receptors, e.g. T-cell receptor and surface immunoglobulin receptor, deficiency in T-cell and B-cell maturation, deficiency in natural killer cell activity, and nonfunctional thymic, lymph node, splenic or bone marrow stroma.

Besides the phenotypic deficiency, further reduction in immunocompetence may be achieved by irradiation of the host, or use of immunocytotoxic labels as indicated previously, e.g. antibodies specific for cells of the lymphoid (including natural killer cells) or myelomonocytic lineages. Particularly, where immunocompetence may be provided by the tissue introduced into the host, native immunocompetence can be enhanced above the low level naturally present in the particular phenotype of the host.

In appropriate situations, one or more organs may be removed for particular purposes. For example, a splenectomy may be performed to provide longer-term reconstitution of circulating red and other hematopoietic cells. Other organs may be removed for introduction and study of a xenogeneic organ. Bone marrow may be removed or destroyed by selective irradiation for introduction of xenogeneic bone marrow. Host stromal cells may be removed to provide xenogeneic stromal cells for a more natural environment for xenogeneic stem cells.

The host will usually be of an age less than about 25% of the normal lifetime of an immunocompetent host, usually about 1 to 20% of the normal lifetime. Generally, the host will be at least about 3 week old and large enough to manipulate for introduction of the mammalian cells at the desired site. For example, mice which may be considered to have about a 2-4 year lifetime are used at about 3 to 10, usually 4 to 8 weeks, of age. Growth of the tissue within the host will vary with the organ, usually being at least a 5-fold, more usually at least a 10-fold and may be a 100-fold or more increase in size (volume).

Depending upon the tissue which is introduced into the host, the nature of the host, and the combinations of tissue employed, various results can be achieved. Of particular interest in the hematopoietic system is the production of sets and subsets of cells, particularly T-cells and/or B-cells. For example, by using fetal liver stem cells in conjunction with a thymus from the same species or host (allogeneic or syngeneic) in the immunocompromised host, T-cells, B-cells, and myelomonocytic cells can be produced which are native to the source host. The production of the T-cells, as well as any other source host cells, may be enhanced by introducing various lymphokines, cytokines, and growth factors from the tissue host source into the mammalian host. In this way, the growth of the desired cells may be further enhanced. Illustrative factors include each of interleukins 1-7, particularly IL-1, -2, and -3; M-, G-, and GM-colony stimulating factor, interferons-$\alpha$, -$\beta$, and -$\gamma$, monokines, growth factors, etc. The amount that may be added will vary depending upon the nature of the mammalian host, the nature of the cell, as well as the nature of the factor.

In order to enhance particular subsets of T- and/or B-cells, the mammalian host may be immunized with an antigen of interest to expand the population of T-cells and B-cells which bind to the particular antigen. The mammalian host may be subjected to extra immunizations to enhance further the desired population. In this manner, B-cells may be produced which are specific for the antigen and may be used as splenocytes, lymph node lymphocytes, or other peripheral blood lymphocytes for fusion with an appropriate fusion partner to produce hybridomas in conventional manners or be immortalized with EBV. Various myeloma cells exist for fusion with primate cells, particularly human cells, such as reported in U.S. Patent Nos. 4,574,116; 4,594,325; and 4,451,570. Alternatively, T-cells may be immortalized to provide for T-cells carrying T-cell receptors which may be used for stimulating B-cells, mixed lymphocyte reactions, evaluating immunodominant sequences, affinity columns for B-cells, particularly in association with an immunodominant sequence, or for cytotoxicity or inflammatory reactions against relevant (e.g. neoplastic) target cells. In addition, various colonies of monocytes, granulocytes, macrophages, eosinophils, neutrophils, myeloid cells, blast cells, precursor cells, and the like may be produced and isolated.

Also, the presence of the foreign (source host) tissue in an immunocompromised host may be used to study the effect of various compounds on the growth, viability, differentiation, maturation, transformation, or the like, of the foreign cells in a live host. Thus, the immunocompromised host may be used to study the effect of variation of a condition on a symptom or indication of a disease. By "condition" is intended a physical chemical or biological property; e.g. temperature, electric potential, ionic strength, drugs, transformation, etc.

Normally, the tissue is vascularized. Thus, various drugs may be administered to the host and the effect on a particular tissue determined by non-invasive or invasive techniques. Non-invasive techniques include NMR, CAT scans, fluoroscopy, roentgenography, radionuclide scanning, ultrasonography, electrocardiography, electroencephalography, evoked potentials, etc. Invasive techniques include biopsy, autopsy, laparotomy, laparoscopy, intermittent intravenous blood sampling, or intravenous catheter, etc. Conveniently, placement of various devices, e.g. catheters, electrodes, etc., may be performed for continuous monitoring. Thus, the host may be used to determine the carcinogenicity of various compounds to different foreign tissues, the effect on growth and viability of various foreign tissues, the effect of combinations of compounds, e.g. drugs, or the like. In addition, by providing for pathogenic infection of the foreign tissue, the effect of various drugs in protecting the host tissue from the pathogen, as well as being cytotoxic to or suppressive of the pathogen in a cellular environment can be determined.

The chimeric host may also be used for evaluating the cytotoxicity of various drugs toward the foreign tissue, for example, for screening for investigative new drug applications. In addition, the chimeric host may be used to evaluate the drugs as to their efficacy, safety and bio-availability.

Organs may be grown for transplantation, so that tissue from the donor host, particularly syngeneic tissue or allogeneic tissue, may be grown to an organ in the mammalian host and then transplanted to a recipient which is of a species able to accept the organ grown in the mammalian host. The cells may be subject to various manipulations, e.g. transfection, to introduce a new phenotype, correct a defective phenotype, etc.

The mammalian host will be grown in conventional ways. Depending upon the degree of immunocompromised status of the mammalian host, the mammalian host may be protected to varying degrees from infection. Thus, in some instances, a sterile environment or prophylactic antibiosis may be indicated. Prophylactic antibiosis may be achieved for SCID mice with 25-75 mg trimethoprim and 100-300 mg sulfamethoxazole in 5 ml of suspension, given 3 days each week. Alternatively, it may be satisfactory to isolate the potential xenogeneic hosts from other animals in germ-free environments after caesarean derivation. The feeding and maintenance of the chimeric host will for the most part follow conventional techniques.

The foreign cells will usually be present for at least two weeks, usually at least four weeks and may be continuously present over periods of three months or more. For the most part, normal cells, tissue, and/or organs may be stably maintained and function for at least three-six months, frequently, at least 10 months.

The foreign cells are capable of remaining viable in the immunocompetent host and will be capable of functioning in the source host and frequently capable of functioning in the xenogeneic host. That is, besides carrying on normal metabolic processes, the cells will respond to ligands, transduce signals, secrete appropriate products and carry on normal functions as carried on by syngeneic or congeneic cells in their wild-type host. Furthermore, where organs are involved, the cells will define a tissue mass with appropriate architecture for the organ function.

The immunocompromised host may be used in a variety of ways associated with its ability to provide an environment in which stem cells may proliferate and differentiate. Thus, the host may be employed to detect the presence of stem cells in a cellular composition, which may be homogeneous or heterogeneous. A cellular composition, such as bone marrow, may be separated into fractions using, for example, a fluorescence activated cell sorter ("FACS"). The fractions may then be injected intravascularly into the host. After sufficient time for the cells to differentiate, tissue or blood may be removed from the host to serve as a source of peripheral blood lymphocytes or other hematopoietic cell, e.g. erythroid, myeloid, and platelets. If necessary, the stem cells could be MHC typed to ensure that the mature cells originate from the stem cells, with the stem cells having different MHC antigens from other hematopoietic cells which may be present in the host. The presence of mature cells of the various hematopoietic lineages would be indicative of precursor cells, while the presence of all lineages would be indicative of stem cells. Again, the FACS could be used with advantage to determine the cellular population.

As illustrative of the subject invention, SCID mice of from about 6 to 8 weeks are employed. Human thymus tissue, spleen, lymph node tissue, and liver tissue are introduced into the renal capsule. Four weeks after implantation, fetal liver tissue cells are injected intravenously. Nine weeks after implantation, the mice are immunized with 2 ml of $10^5$ cells of live E. coli J5 organisms in complete Freund's adjuvant, which organisms due to a mutation have the core lipid A exposed. Eleven weeks after implantation, a second immunization of $10^5$ cells of E. coli J5 in incomplete Freund's adjuvant is administered. Three days later, the mice are exsanguinated, the PBL, and/or lymph node and/or spleen cells of human origin are fused with a heteromyeloma described in U.S. Patent No. 4,574,116, as described therein. The hybridomas are expanded, cloned under limiting dilution, and the clones screened for antibodies to lipid A core. The resulting human antibodies may be screened for protection against bacteremia and anaphylactic shock.

Another example is the use of the chimeric host for studying disease. For example, a chimeric mouse into which human fetal liver tissue, thymus tissue and lymph node tissue have been introduced may be employed for study of HIV. After a period of four weeks after introduction of the fetal liver tissue, when the level of T-cells has been approximately maximized, the mouse is infected with an intravenous dose of $10^4$ infectious units of HIV. One week later a biopsy is performed to ensure that infection has occurred. The mouse may then be used to screen therapeutic agents for HIV. For example, 5-50 mg/kg of host anti-CD4 is added as a daily dosage over a period of 3 days to a week, following the course of infection by serial biopsy. Antibodies which reduce the level of infection are then used to vary the course of administration prior to use in human hosts.

The following examples are offered by way of illustration and not by way of limitation.

EXPERIMENTAL

Methods

1. Mice.

C.B17 scid/scid mice were obtained from Dr. Leonard D. Shultz of The Jackson Laboratory, Bar Harbor, ME. The mice are housed in standard isolator cages within a routine animal holding facility. Under these conditions, they have a lifespan that is considerably shorter than that of other inbred immunocompetent strains (e.g., 1-2 yrs vs. 3-4 yrs). The cause of death is normally related to opportunistic infection (most often by Pneumocystis carinii). Protocols to prevent such infections by administering prophylactic antibiotics (trimethoprim/sulfamethoxasole) to the mice are being employed (see above) using as guidelines protocols developed for the prophylaxis of patients with AIDS or ARC. In all other respects (e.g. bedding, food, daily light cycles, etc.), the mice are handled as per routine animal holding facility protocols.

EP 0 322 240 B1

## 2. Collection and preparation of human fetal tissues.

The information that is known about the patient includes the approximate (or where known, the actual) gestational age of the fetus and the given reason for the abortion; in the latter case, also known are the details of any genetic or morphologic anomaly discovered by amniotic fluid analysis and/or ultrasonography (e.g., chromosomal defects, anencephaly, hydrops, etc.). In initial experiments, tissue from fetuses that are apparently normal were used; in later experiments, specifically constructed situations were created in which tissues from fetuses with genetic anomalies were tested.

The tissues are obtained directly in the operating room as fetal parts after elective or medically-indicated abortion (with gestational ages ranging from 7-24 weeks). Without maintaining strict sterility, these parts are taken immediately to a gross dissection room. The desired tissues are identified, dissected out, placed into RPMI 1640 medium with 10% fetal calf serum, and transported directly to another lab. In those situations in which "whole organ" transplants (i.e. tissue inclusive of both the stromal elements and of the hematopoietic elements therein) are being performed, the relevant organs are cut into sections that are approximately 1 mm by 4 mm. (A piece of tissue of this size fits easily into the 19-gauge trocar that is used for implantation.) In those situations in which dispersed cells are to be injected, the relevant organs are teased apart to yield viable cells in suspension. In the case of fetal liver, the suspension cells are enriched for hematopoietic precursors by ficoll-hypaque density gradient centrifugation; the interface layer containing the desired cells is then washed 3x, counted, and brought to approximately $10^8$ cells/ml. For subfractionation of stem cell precursors, fetal liver cells isolated on ficoll-hypaque gradients are subsequently stained with monoclonal antibodies against relevant cell surface markers and isolated by techniques including negative selection with magnetic beads and positive selection on the FACS.

To mark the genetic origin of the donor fetal tissue, HLA histotyping is performed on fetal thymocytes using monoclonal antibodies (see below) that recognize common HLA alleles. In this manner, prior to implantation, a histotyped "fingerprint" is obtained by which all subsequent progeny of the introduced stem cells may be specifically followed in the SCID-hu mouse.

Tissue is normally introduced in as fresh a state as possible. Therefore, the tissue collection, preparation, histotyping, and implantation are done all on the same day. Frozen tissue, however, appears to work well in the case of fetal liver cells and fetal thymus tissue. Therefore, aliquots of remaining tissue from each specimen are frozen down at the end of the day 10%DMSO/50%FCS using standard procedures and then catalogued in a liquid nitrogen storage freezer.

## 3. Transplantation of human tissue into SCID mice.

Mice have generally been used at 4-8 weeks of age, either without pretreatment or after pretreatment with either (a) anti-asialoglycoprotein GMI or (b) fractionated courses of irradiation (175 rads weekly x 4 weeks). The latter two pretreatment regimens have been designed to test the possibility that removal of endogenous natural killer cell activity in the SCID may permit better constitution with xenogeneic tissue.

Tissue has been introduced by a number of routes: intravenously, intrarenally, intrasplenically, or subcutaneously. In the latter three cases, the mice are first anesthetized with halothane. A 1 cm incision is made to expose either the kidney or the spleen and a 19 g trocar is used to introduce human tissue beneath the capsule of either organ. Thereafter, the incision is approximated with surgical sutures. For intravenous injections, a 30 g needle is used to introduce suspension cells into the retro-orbital venous plexus. Of these routes, implantation of tissue into the left kidney capsule affords several distinct advantages: first, it is easily accessible; secondly, the kidney may be exposed repeatedly to observe the growth of the transplanted tissue and to remove biopsy specimens from it for histologic analysis.

## 4. Analysis of SCID-hu mice.

### A. Immunophenotyping.

A number of monoclonal antibodies against various cell surface markers of human lymphocytes have been prepared for the analysis of cells found within SCID/Hu mice. These include markers of mature human peripheral blood T-cells (OKT4, OKT8, OKT3, OKT11), markers of human peripheral blood B-cells (anti-IgM, anti-IgG, anti-light chain), and markers of the human HLA complex (including both polymorphic and monomorphic determinants of Class I antigens). These murine antibodies have been used in immunofluorescence assays, either as primary antibodies that are secondarily reacted with a fluoresceinated (FITC) anti-mouse antibody, or as biotinylated antibodies that are secondarily reacted with avidin-FITC. In

8

some instances, antibodies that are directly fluoresceinated have also been used. After transplantation, mice are bled weekly by tail vein incision; approximately 100 $\mu$l of whole blood is obtained (usually containing 1-2 x $10^5$ cells). Nucleated cells are enriched by precipitation of red blood cells with dextran sulfate, washed free of platelets, and then stained with the monoclonal antibodies within 96-well microtiter plates. After the assay is complete, a visual inspection for positive cells (that is, those with unambiguous surface fluorescence) is made under the immunofluorescence microscope. If human cells represent greater than 1% of the total nucleated cells in the periphery, they may be readily detected by this method and also quantitated with statistical significance with a multiple parameter FACS. Accordingly, if the samples are positive by direct inspection, they are then analyzed by FACS. In this analysis, the cells are fixed first with 1% formalin and then incubated for 10 min with 1 $\mu$g/ml propidium iodide (PI) (which is taken up by nucleated cells). In subsequent FACS analyses, cells are first gated for PI (that is, they are nucleated) and then assessed for staining with FITC. In this manner, the percentage of FITC-positive cells as a function of total nucleated cells may be readily obtained.

B. Histology.

At various times post-transplantation, tissues are taken either by biopsy (of, e.g., the tissue growing beneath the left renal capsule) or by autopsy and preserved in paraffin for thin-section. Thereafter, sections are stained with the above monoclonal antibodies and counterstained with a secondary alkaline phosphatase-labelled secondary antibody. The presence of cells positive for the marker is then detected with the alkaline phosphatase reaction product of diaminobenzidine and visualized under light microscopy.

C. DNA analysis.

Cells taken by tailvein bleed or by resuspension of organ systems after biopsy or autopsy are treated by standard methods for the extraction of total cellular DNA. Total cellular DNA derived from SCID-hu mice has been analyzed using various probes and assay systems and compared in parallel to DNA obtained from untreated SCID mice or from normal human controls. The probes include the following: 1) Alu repeat (BLUR 8), a repetitive sequence of DNA found in the human genome but not homologous enough to murine sequences to cross-hybridize under the conditions used in these experiments, 2) human T-cell receptor (B chain) probes, 3) human Ig constant region probes, 4) human MHC Class I and Class II probes. For use in dot blots and in Southerns (run under standard conditions), these probes are first labelled with $^{32}$P by the hexamer-labelling technique. Rearrangements in the T- and/or B-cell receptor genes are sought by restriction endonuclease digestion of total cellular DNA, using known restriction maps and standard conditions. For polymerase chain reactions, using the thermostabile Taq 1 polymerase, a number of oligonucleotides have been constructed and/or obtained, including those to generate copies of the Alu repeat as well as of the Class I and Class II monomorphic sequences.

D. Karyotype analysis.

For karyotype analysis, cells taken by tail bleed have been put into culture with phytohemagglutinin to induce proliferation of human cells. At 48 hours, metaphase arrest is induced by the addition of colchicine and chromosomes prepared for analysis by light microscopy.

Results

1. Human fetal thymus grows in SCID mice.

The growth of human fetal thymus has been observed in most (>80%) SCID mice that have been transplanted with human fetal thymus (including >300 mice in successive experiments at various times over about a 18-month period). In those cases where thymus growth is not observed, problems related to implantation technique are likely to be the cause. The gross architecture is observed by re-opening the flank incision initially made to implant the thymic specimens and exposing the kidney. By 4-8 weeks post-implantation, marked increase in size is almost invariably evident (in very approximate terms ranging from a two-fold to a 20-30 fold increase.) In several cases, fragments of human fetal thymus implanted into one mouse have been removed and transferred to a second, previously untreated mouse. In these cases as well, dramatic growth of thymic tissue has been subsequently observed. The thymic tissue is similar to that of normal human fetal thymus in both color and in consistency. A well-defined vascular system may be observed with low-power magnification. When biopsy specimens are prepared for tissue section and stained with monoclonal antibodies against antigens present on human or murine cells, it is found that the cellular contents of the implanted thymus are mostly human (see below) and that the microscopic anatomy of the tissue has been generally conserved. Thus, well-defined medullary and cortical compartments are present, with characteristic staining of epithelial components with the antibodies MD1 and CDR2, respectively. Dendritic-like cells positive for human Class II determinants are found in the medulla and, to a lesser degree, in the cortex. Thymocytes which stain for the human T-cell markers CD3, CD4, CD8, and CD2 are also present. When analysed by dual-laser FACS, the percentage of cells that are double-positive for CD4 and CD8, single-positive for CD4 or CD8, or double-negative for both markers is similar to that found in a normal, age-matched fetal thymus. The above findings have been made with mice that have received thymic tissue from human fetuses aged 9-22 gestational weeks.

The only difference noted to date between the SCID-hu thymus implant and a normal human fetal thymus is the presence, in the former but not in the latter, of dendritic-like cells in the cortex and (to a lesser degree) in the medulla which express murine Class II determinants. These cells are likely derived from murine, bone-marrow precursors which migrate into the vascularized SCID-hu thymus implant.

A number of important conclusions may be drawn from the above observations. First, human fetal thymus tissue is not rejected by the SCID mouse. Indeed, it is richly vascularized and grows instead. Secondly, by gross morphology and microscopic anatomy, the implanted human thymic tissue bears close resemblance to its normal human counterpart. Thus, the organ appears to maintain intrinsic properties for spatially correct regeneration.

Also, the number of and relative subset distribution of thymocytes appears to be normal. Finally, murine dendritic cells bearing murine class II determinants are found in the SCID-hu thymus. These cells may be involved in "teaching" the developing human thymocytes to be tolerant of (and possibly MHC-restricted to) murine MHC antigens. Thereby, graft-versus-host disease may be averted.

2. Phenotypicaly mature human T-cells are found in the peripheral circulation of SCID/Hu mice.

This phenomenon has been observed in >300 sets of experiments. By FACS analysis using monoclonal antibodies against human T-cell markers it is predictably time-dependent, occurring 4-12 weeks after intravenous introduction of human fetal liver cells into a SCID mouse previously (or simultaneously) implanted with human fetal thymus.

Specifically, sets of mice received human fetal thymus implants. These sets were subdivided into groups: some received $10^7$ fetal liver cells (FLC) intravenously at the same time, some received $10^7$ fetal liver cells intra-thymically two weeks later, some received no fetal liver cells at all. At three weeks, the peripheral blood of each was tested for the presence of cells staining for the human T-cell markers CD3, CD4, CD8, CD2; all were negative. By 4-12 weeks, however, such cells were visible on FACS (constituting 2-40% of the total mononucleated cell population). The ratio of $CD4^+$ to $CD8^+$ human T-cells in SCID-hu peripheral circulation during this time was between 3-4 (mean 3.5, n = 85, S.D. = 0.86), well within the normal range. Tested again between 12-15 weeks post-FLC transplantation, cells bearing these markers were no longer present. This general pattern was true irrespective of the route by which FLC cells were initially administered.

From this analysis it appears to take 4 weeks for significant numbers of cells bearing markers of human T-cells to appear in the peripheral blood of SCID-hu mice. Before that time they are not present; after 12 weeks, they disappear. A corollary is that immunophenotyping is probably legitimate; that is, the antibodies that were used are only reactive in a time-dependent manner and appear not to bind indiscriminately to murine cells. Of interest, the transient wave of human T-cells in the periphery may occasionally be observed whether or not fetal liver cells are given. This appears to mean that cells that are resident within the fetal thymus, at the time that it is implanted, are also able to exit the thymus and to enter the peripheral circulation.

After it had been determined that peripheral blood cells bearing human markers were absent from the SCID-hu animals, all were once again reconstituted with FLC; notably, all of these animals retained the original human fetal thymus implants. In this second round of reconstitution, the animals were once again subdivided into groups: some received graded doses of FLC cells intravenously (ranging from $5 \times 10^6$ to $5 \times 10^7$); other received fragments of whole fetal liver, given both subcutaneously and intrasplenically. Analysis of peripheral blood 3 weeks later revealed the complete absence of cells bearing human markers. By 6 weeks, on the other hand, all animals once again showed the presence of significant numbers of cells bearing the human markers CD3, CD4, CD8 and CD2 (approximately 4-30% of total mononuclear cells); notably 3-15% of these cells expressed the CD4 marker. When two mice were subjected to autopsy one week later (week 7), both showed cells in the periphery that stained either with OKT4 (a marker of the T helper cell lineage) or with OKT8 (a marker of the T cytotoxic/suppressor cell lineage(s)); the ratio of the two respective subpopulations was 2.3:1, well within the range of the normal ratio found in human peripheral blood itself. To ascertain whether these cells might actually home to the murine spleen, thymus, or lymph node, FACS analysis was also performed on cells obtained from these organs. In these cases, no cells bearing the human T-cell markers were observed. In subsequent FACS analyses of peripheral blood, all but one set of mice were observed to once again lose the peripheral population of human T-cells.

When SCID mice are implanted with whole fetal liver fragments and fetal thymus, and then given an intravenous injection of fetal liver cells, the same observations are made with a critical difference. Instead of lasting only 12 weeks, the human T-cells in the peripheral circulation are found to last at least 40 weeks. Again, the CD4/CD8 ratio is normal throughout.

This second round of reconstitution prompts a number of important conclusions. First, the phenomenon of a transient wave of human cells in the peripheral blood of SCID-hu mice given FLC intravenously is reproducible. Secondly, since the FLC were given intravenously and since they subsequently showed markers of mature T-cells, it is likely that they actually homed to and through the implanted human fetal thymus. Thirdly, the mature T-cells found in the periphery express a "physiologic" balance of CD4/CD8 subpopulations. Fourthly, it is apparent that these peripheral human T-cells do not actually home to the resident murine organs (spleen, lymph node, thymus). Thus, in ongoing experiments, the human equivalents have been implanted into SCID-hu mice. Finally, the introduction of whole fetal liver is found to generate a prolonged wave of human cells in the periphery. This observation implicates the presence of a self-renewing stem cell population within FLC, which is unable to replicate in the absence of fetal liver stromal cells.

Notwithstanding the apparent specificity of the monoclonal antibodies against human mature T-cell markers (note, even in those mice that showed high percentages of staining cells in the peripheral blood, spleen cell populations analyzed at the same time were negative), it remained a formal possibilty that the staining was due to an unforeseen artifact. To control for this possibility, DNA analysis of the cells found in the peripheral blood was performed using a probe specific for human, but not murine, repetitive DNA sequences (the ALU repeat: BLUR-8). By this analysis, it was found that the peripheral blood cells derived from the SCID-hu mouse not only bear the phenotypic markers characteristic of human T-cells, but also contain DNA that strongly hybridizes with the human ALU probe.

When SCID mice are implanted with human fetal thymus and lymph node, and then given an intravenous injection of human fetal liver cells, human IgG may also be detected in the serum. The amount of IgG varies between 5-10% of that found normally. The plasma cells producing the IgG can be localized to the implanted human lymph node. Human IgG production requires at least the introduction of human fetal liver cells and human lymph node into SCID mice.

In a specific experiment SCID mice were maintained in bonneted isolation cages and received TMS in suspension through the drinking water for 3 days of each week (40 mg of trimethoprim and 200 mg of sulfamethoxazole per 5 ml of suspension; 0.125 ml of suspension per 4 ml of drinking water per mouse per day). The drinking water bottle was turned daily while the drug was being administered; standard drinking water was substituted for the remaining 4 days of each week. A 9 g.w. human fetal thymus lobe 0.5 by 0.5 by 2mm in size and a 0.5 by 0.5 by 2 mm segment of a human lymph mode were implanted under the left kidney capsule. These organs were obtained from an HLA-A2$^+$, HLA-B7$^-$ donor. Simultaneously, the mouse had received $10^7$ liver cells intravenously; these cells, in contrast, were HLA-A2$^-$, HLA-B7$^+$.

The fetal liver cells were obtained by mincing and cells remaining suspended in RPMI 1640 medium with 10% FCS were separated into a mononuclear fraction by ficoll-hypaque centrifugation; the interface cells were then washed 3x in RPMI 1640 with 10 percent FCS and resuspended at a concentration of $10^8$ cells/ml for intravenous administration.

For implantation of tissue, mice were first anesthetised with halothane. A 1-cm flank incision was made to expose the left kidney. Sutures were placed to approximate successive peritoneal and fascial layers and metal clips were secured over the wound to ensure healing. Suspended fetal liver cells were injected intravenously by a retro-orbital approach with a 30-gauge needle.

Frozen serial fixed sections of the thymus four weeks after implantation were compared to a normal age-matched fetal thymus. The microscopic anatomy was found to be comparable, with the exception of murine class II-positive cells having a dendritic morphology being present. In particular, all of the thymocytes were HLA-A2$^-$, HLA-B7$^+$; therefore, they were derived from stem cells within the FLC preparation given intravenously.

When peripheral blood from the SCID-hu mice were analyzed, human T-cells with a CD4/CD8 ratio of 3.5 were found between weeks 4-12. These cells expressed the antigen HLA-B7, but not HLA-A2; therefore, they were fully differentiated cells from the original FLC source, having been processed first through the HLA-A2$^+$, HLA-B7$^-$ thymus.

To better determine which of the various FLC subpopulations actually give rise to the mature human T-cells (e.g., which subpopulation(s) possessed stem cell activity), FLC from an HLA-A2$^-$, HLA-B7$^+$ donor were depleted of CD4$^+$, CD8$^+$, M1$^+$, M3$^+$, Ig$^+$, A$^+$/B$^+$ cells (removing T, B, myelomonocytic, erythrocytic, and granulocytic cells) to produce a "lineage-marker minus" (lin$^-$) population. This population was enriched by FACS into two subpopulations with an antibody against human Thyl: lin$^-$ thyl$^-$ and lin$^-$ thyl$^+$. These populations were then introduced intravenously into SCID mice with a HLA-A2$^+$, HLA-B7$^-$ human thymus implant. The lin$^-$ thyl$^+$ subpopulation was found to give rise to HLA-B7$^+$ T-cells later; the lin$^-$ thyl$^-$ population was inactive. Therefore, the lin$^-$ thyl$^+$ population is likely to be inclusive of human hematopoietic stem cell activity.

Finally, when serum specimens from the SCID-hu mice were analyzed, they were found to contain human IgG at levels of 1-10 mg/ml. Human lymph node biopsies showed the presence of human IgG-containing plasma cells by immunohistochemical stain.

In another study, the ability to cause lymphotrophic infection with a human-specific pathogen was studied. Particularly, an HIV-1 isolate was employed for infection, where the isolate was found to be infectious in vivo, but not in vitro.

A molecularly-cloned isolate of HIV-1 was used. Derived from the cell-free cerebrospinal fluid of a patient with subacute encephalopathy and Kaposi's sarcoma, HIV-1$_{JR-CSF}$ was propagated first in short-term culture with phytohemagglutinin-stimulated human peripheral blood lymphocytes in vitro and then molecularly cloned. This isolate has found to be infectious for mitogen-stimulated human T-cell blasts and for glial cell explants, but not for primary monocyte/macrophage cultures. It has not been passaged, and does not grow, in continuous human T or monocyte/macrophage cell lines. Such tropism marks a potentially important difference between it and other isolates which have been molecularly cloned and adapted to growth in vitro (e.g., HXB2, HTLV-III$_B$, ARV, and LAV).

A flank incision was made to expose the growing human thymus or lymph node implant of anesthetized SCID-hu mice. Graded doses of HIV-1$_{JR-CSF}$ were introduced by direct intra-thymic or intra-nodal inoculation. The mice were then maintained within micro-isolator cages, inside a glove box. At varying time points, biopsy specimens of the infected lymphoid organs were prepared, fixed in 4% paraformaldehyde, and assayed for signs of acute infection.

Infection of SCID-hu mice with HIV-1$_{JR-CSF}$ resulted in easily detectable signs of viral replication. The most directly informative assay has been in situ hybridization of infected tissue sections with the RNA probe pG4. This probe hybridizes to the 3′ end of the genomic HIV-1 transcript; accordingly, all viral messages are detected without discrimination. Under the conditions of the assay, viral DNA is not detected. The results indicate that infection of the human thymus and lymph node implants is time-dependent. At one week after direct intra-thymic inoculation (of 400-4000 infectious units), no cells in the section wre seen to hybridize with the pG4 probe. At 2, 4, and 8 weeks, progressively increasing numbers of cells were found to hybridize (see Table 1). This suggests that ongoing rounds of infection occurred in vivo. The infected cells were scattered throughout the cortex and medulla of the injected human thymus and diffusely across the injected human lymph node. Discrete foci of infection, involving more than one contiguous cell, were only rarely observed. Possibly, within a given tissue plane, contiguous cells may be either resistant to infection or infected at an undetectable level. Alternatively, infected cells may disseminate from another focus, not included in the section (e.g., from migrating progenitor cells, infected elsewhere).

TABLE 1

QUANTITATION OF INFECTION BY HIV-1
IN THE SCID-HU THYMUS

Week Post-Infection

| | 1 | 2 | 4 | 8 |
|---|---|---|---|---|
| **Medulla** | | | | |
| ISH+ | 1 | 30 | 50 | 116 |
| IH+ | ND | 4 | 30 | 31 |
| % +/+ | -- | 10 | 60 | 29 |
| **Cortex** | | | | |
| ISH+ | 1 | 12 | 6 | 24 |
| IH+ | ND | 0 | 2.5 | 7 |
| % +/+ | -- | 0 | 45 | 31 |

ISH - in situ hybridization
IH  - immunohistochemistry - viral protein (GB)

Results expressed is the number of infected cells,
cells detected by ISH or IH, in 10 independent high
power (200X) fields. Read by two observers.

The events described are specific for HIV-1 infection. Prior heat-inactivation of virus (80°C, 1 hr) destroyed infectivity. No cells in infected tissue were found to hybridize with pG4 RNA probes in the "sense" orientation. No cells in uninfected tissues were found to hybridize to pG4 probes in either orientation. Finally, the infection is not only time-dependent but also dose-dependent. Thus, a $10^{-1}$ dilution of HIV-1$_{JR-CSF}$ was found to be infectious, but dilutions of $10^{-3}$-$10^{-5}$ were not. Using estimates of viral titers determined by in vitro assays, the $10^{-1}$ concentration corresponds to 400-4000 infectious units.

The above analysis was expanded by applying immunohistochemical stains to the tissue sections, prior to in situ hybridization. In this manner, the presence of viral protein could be detected in cells making viral RNA transcripts; in addition, the cell surface phenotype of infected cells could be determined. In this case, a polyclonal antiserum (GB) reactive against gag, pol, and env epitopes was used to analyze sections of human thymus, two weeks after infection with HIV-1$_{JR-CSF}$. More cells were found to be infected in the medulla than in the cortex, even though there was an abundant representation of CD4+ thymocytes in each compartment. A similar situation was found at weeks 4 and 8 post-infection, throughout multiple planes of observation. Thus, within any given section at any given time, 70-90% of the infected cells were medullary in location. Rarely, infected multinucleated cells, perhaps representing syncytia, were seen.

In both the medulla and the cortex of the thymus, as well as in the lymph node, two distinct modes of viral replication were apparent by combination immunohistochemistry and in situ hybridization. In one mode, cells were found to make detectable amounts of viral protein and RNA; in another, only viral RNA transcripts were found. The latter population represents the majority of the total infected cells in each compartment (Table 1). The basis for this observation is not clear. An artifact related to the procedure itself appears unlikely, cells expressing each mode of replication are randomly dispersed and frequently adjacent. Rather, those cells which are not stained with the antiserum GB may be making viral protein at levels below the level of sensitivity for detection; alternatively, they may be making different viral proteins (e.g., with epitopes not detected by the various antibodies in the serum).

In either case, disparate patterns of transcriptional and/or translational control post-infection must be invoked. Since the results are obtained from infection with a molecular clone of HIV-1, it is likely that unique cellular environments dictate the differences in control. These various environments may be inclusive of distinct cell lineages and of successive steps of activation or differentiation within given lineages. Complex patterns of this sort have been documented in vivo after infection with other lentiviruses (e.g., visna, canine

EP 0 322 240 B1

arthritis-encephalitis virus). Their existence is consistent with recent experiments on HIV-1 in vitro. The regulatory mechanisms by which they occur may now be explored with HIV-1 in vivo, within the SCID-Hu mouse.

It is evident from the above results, that the subject invention provides for a wide gamut of opportunities due to the ability to grow tissue in a xenogeneic mammalian host, where not only can individual organs be grown, but also cells and tissue from different organs can be grown, where the cells and/or organs may interact, such as the hematopoietic system, the endocrine system, and the like. Because of the ability to grow the foreign tissue, the effect of various substances on the tissue and the interaction of different tissue can be determined in an environment which to a significant degree approximates the normal environment of the tissue. Furthermore, the hematopoietic tissue allows for immortalization of cells produced in the mammalian host. Stem cells giving rise to the hematopoietic system may be isolated by FACS and shown to be active. They may then be grown in vitro with known growth factors. Alternatively, they may be used to help identify unknown factors involved in their self-renewal. Monoclonal antibodies that were formerly difficult to obtain, particularly human monoclonal antibodies, may be readily obtained by immunization of the mammalian host with a variety of antigens, which would not normally be permitted to be used in a human host. In this manner, the repertoire of human monoclonal antibodies may be greatly expanded for the treatment of various diseases, including infection from pathogens, neoplastic conditions, and the like. In like manner, differentiated, antigen-specific, MHC-restricted or unrestricted human T-cells may be isolated for expansion in vitro and later therapeutic use. Furthermore, organs may be used which may be transplanted into an allogeneic host of the tissue source.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

**Claims**

1. A chimeric non-human animal host comprising:

an immunodeficient animal host comprising at least a portion of an immune system, lacking functional syngeneic lymphocytes but in which the thymus is present; and

xenogenic cellular material, being a xenogenic organ or tissue capable of functioning in the host, or xenogenic cells together with, if required for the maturation of the cells, such an organ or tissue, the xenogenic cellular material being introduced into the animal host at an age of at least neonate.

2. A chimeric animal host according to claim 1, wherein said animal host is immunodeficient due to a genetic defect in lymphoid cells.

3. A chimeric non-human animal host comprising:

an immunodeficient animal host comprising at least a portion of an immune system, lacking functional syngeneic lymphocytes but on which the thymus is present; and

wherein at least one organ comprises a xenogeneic functional thymus and optionally xenogeneic cells, tissues and/organs, said cells comprising at least one of hematopoietic stem cells, immature differentiated hematopoietic cells or mature differentiated hematopoietic cells, as a result of introduction of said xenogeneic cells tissue and/or organ into said animal host at an age of at least neonate.

4. A chimeric animal host according to claim 3, wherein said at least one organ is capable of functioning from the time of introduction into said host for at least three months.

5. A chimeric animal host according to Claim 3, wherein said animal is a mammal.

6. A chimeric non-human mammalian host comprising:

an immunodeficient mammalian host, lacking functional syngeneic lymphocytes but in which the thymus is present; and

xenogeneic cells and at least one organ or progenitor thereof, wherein said at least one organ comprises a functional thymus and said cells comprise at least one of hematopoietic stem cells,

14

immature differentiated hematopoietic cells or mature differentiated hematopoietic cells, as a result of introduction of said xenogeneic cells and organ or progenitor thereof into said animal host at an age of at least neonate.

7. A chimeric mammalian host according to Claim 6, wherein said at least one organ further comprises at least one of lymph node, liver tissue or progenitor thereof.

8. A chimeric mammalian host according to Claim 6, wherein said xenogeneic cells are human cells and said organ is comprised of human cells.

9. A chimeric mammalian host according to Claim 8, wherein said mammalian host comprises mature differentiated human hematopoietic cells as a result of maturation in vivo of said stem cells introduced into said mammalian host.

10. A chimeric mammalian host according to Claim 9, wherein said stem cells are from a human host at an age of at least neonate.

11. A chimeric mammalian host according to Claim 6, wherein said xenogeneic cells and organ are human and fetal when introduced into said mammalian host.

12. A chimeric mammalian host according to Claim 11, wherein said mammalian host comprises human mature peripheral blood lymphocytes as a result of maturation in vivo of said stem cells introduced into said mammalian host.

13. A chimeric mammalian host according to Claim 12, wherein said xenogeneic cells or organ is infected with a pathogen.

14. A chimeric severe combined immunodeficient mouse host comprising:
xenogeneic cells and at least one organ or progenitor thereof, wherein said at least one organ comprises a functional thymus and said cells comprise at least one of hematopoietic stem cells, immature differentiated hematopoietic cells or mature differentiated hematopoietic cells, as a result of introduction of said xenogeneic cells and organ or progenitor thereof into said mouse host at an age of at least neonate.

15. A mouse host according to Claim 14, wherein said at least one organ further comprises at least one of lymph node, liver tissue or progenitor thereof.

16. A mouse host according to Claim 14, wherein said xenogeneic cells are human cells and said organ is comprised of human cells.

17. A mouse host according to Claim 16, wherein said mouse host comprises mature differentiated hematopoietic cells as a result of maturation in vivo of said stem cells introduced into said mouse host.

18. A mouse host according to Claim 17, wherein said differentiated hematopoietic cells comprise peripheral blood lymphocytes.

19. A mouse host according to Claim 14, wherein said stem cells are from a mammalian source of an age of at least neonate.

20. A mouse host according to Claim 14, wherein said xenogeneic cells and organ are fetal when introduced into said mouse host.

21. A mouse host according to Claim 20, wherein said mouse host comprises human mature differentiated hematopoietic cells as a result of maturation in vivo of said stem cells introduced into said mouse host.

22. A mouse host according to Claim 21, wherein said xenogeneic cells or organ is infected with a pathogen.

EP 0 322 240 B1

**23.** A mouse host according to Claim 22, wherein said pathogen is HIV.

**24.** A mouse host according to Claim 21, wherein said at least one organ further comprises at least one lymph node or progenitor thereof and said mouse host further comprises human antibodies.

**25.** A method for producing human plasma cells, said method comprising:

growing a chimeric severe combined immunodeficient mouse host comprising:

human cells, at least two organs, wherein said at least two organs comprise a functional thymus and a functional lymph node and said cells comprise at least one of hematopoietic stem cells, immature differentiated hematopoietic cells or mature differentiated hematopoietic cells, as a result of introduction of said xenogeneic cells and organ or progenitor thereof into said mouse host at an age of at least neonate, and an antigen recognized by said mature differentiated hematopoietic cells, whereby plasma cells are produced.

**26.** A method for producing human
immunoglobulin, said method comprising:

isolating plasma cells or a progenitor thereof from a chimeric severe combined immunodeficient mouse host comprising:

xenogeneic cells, at least one organ, wherein said at least one organ comprises a functional thymus and said cells comprise mature differentiated hematopoietic cells, as a result of introduction of said xenogeneic cells and organ or progenitor thereof into said mouse host at an age of at least neonate, and plasma cells as a result of the presence of an antigen recognized by said mature differentiated hematopoietic cells;

immortalizing said isolated plasma cells or progenitor thereof to provide an immortalized cell which secretes immunoglobulin; and

screening said immortalized cells for production of immunoglobulin.

**27.** A method for determining the effect of a variation of a condition on disease symptoms or indications caused by a diseased state in a mammalian host, said method comprising:

causing a diseased state in a chimeric severe combined immunodeficient mouse host comprising:

xenogeneic cells, at least one organ, wherein said at least one organ comprises a functional thymus and said cells comprise at least one of hematopoietic stem cells, immature differentiated hematopoietic cells or mature differentiated hematopoietic cells, as a result of introduction of said xenogeneic cells and organ or progenitor thereof into said mouse host at an age of at least neonate, and a second organ when said disease symptom or indication is associated with such second organ;

subjecting said mouse host to said variation; and

determining the effect of said variation on said disease symptom or indication.

**28.** A method according to Claim 27, wherein said variation is administration of a drug.

**29.** A method according to Claim 27, wherein said variation is administration of lymphocytic or myelomonocytic cells specific for said diseased state.

**30.** A method for producing a chimeric host capable of processing xenogeneic stem cells, said method comprising:

introducing into a severe combined immunodeficient mouse, at a site at which xenogeneic cells can grow, fetal thymus tissue and at the same or different site, xenogeneic stem cells.

**31.** A method according to Claim 30, including the additional step of introducing at least one of xenogeneic lymph node tissue or liver tissue.

**32.** A method according to Claim 31, wherein said xenogeneic cells and tissue are human cells and tissue.

**33.** A method according to Claim 31, wherein said fetal thymus tissue is of an age of about 9 to 24 gestational weeks and said lymph node tissue is of an age of greater than about 15 gestational weeks.

**34.** A method according to Claim 33, wherein said xenogeneic cells and tissue are human cells and tissue.

16

EP 0 322 240 B1

**35.** A method according to Claim 30, wherein said site for said thymus tissue is the kidney capsule.

**36.** A method according to Claim 30, wherein said mouse host is defective in a physiological function other than the hematopoietic system.

**37.** A method for detecting the presence of stem cells in a composition comprising a mixture of cells, said method comprising:
dividing the cells into fractions based on surface membrane protein markers;
introducing at least one of said cellular fractions into a mouse host according to Claim 14;
allowing said stem cells to mature in said mouse host;
removing a source of peripheral blood lymphocytes from said mouse host; and
determining the presence of differentiated cells from said stem cells as indicative of said stem cells in said fraction.

**38.** A method for producing a subset of human hematopoietic cells, said method comprising:
introducing human hematopoietic stem cells into a mouse host according to Claim 14, with at least one lymphokine for causing differentiation into at least one of the hematopoietic lineages;
maintaining said hosts for sufficient time for said stem cells to differentiate and proliferate to produce cells of said subset; and
harvesting said subset of human hematopoietic cells.

**39.** A method of producing modified human cells including subjecting human cells to the environment of a chimeric immunodeficient non-human animal host comprising at least a portion of its immune system, lacking syngeneic lymphocytes but in which the thymus is present, without subjecting cells of the host mammal to the said modification.

**40.** A chimeric immunodeficient non-human mammal including human cells, the human cells being specifically modified as a result of the environment in the mammal, the mammal having at least a part of its immune system, lacking syngeneic lymphocytes but in which the thymus is present.

**41.** A method or mammal according to Claim 39 or Claim 40 wherein the cells are present as part of an organ.

**42.** A method or mammal according to Claim 39, Claim 40 or Claim 41 wherein the human cells are lymphocyte cells and the modification in the cells is an immune response.

**43.** A method or mammal according to Claim 39, Claim 40 or Claim 41 wherein the human cells are a target for an injection agent and the modification is an injection of the cells.

**44.** A method or mammal according to Claim 43 additionally including a therapeutic agent contacting the injected cells.

**45.** A mammal as claimed in any one of Claims 40 to 44 wherein the human cells are from or are the lymph node and the mammal is for use in testing for HIV.

**Patentansprüche**

**1.** Chimärer, nicht-menschlicher, tierischer Wirt, umfassend:
einen immunodefizienten, tierischen Wirt, umfassend zumindest einen Abschnitt eines Immunsystems, dem funktionelle syngene Lymphozyten fehlen, in dem aber der Thymus vorhanden ist; und
xenogenes Zellmaterial, das ein xenogenes Organ oder Gewebe ist, das im Wirt funktionsfähig ist, oder xenogene Zellen zusammen mit, falls zum Reifen der Zellen benötigt, einem derartigen Organ oder Gewebe, wobei das xenogene Zellmaterial in den tierischen Wirt mit einem Alter von zumindest neugeboren eingebracht wird.

**2.** Chimärer, tierischer Wirt nach Anspruch 1, worin der tierische Wirt aufgrund eines genetischen Defekts an Lymphoidzellen immunodefizient ist.

3. Chimärer, nicht-menschlicher, tierischer Wirt, umfassend:

einen immunodefizienten, tierischen Wirt, der zumindest einen Abschnitt eines Immunsystems aufweist, dem funktionelle syngene Lymphozyten fehlen, in dem aber der Thymus vorhanden ist; und

worin als Ergebnis des Einbringens der xenogenen Zellen, des Gewebes und/oder des Organs in den tierischen Wirt mit einem Alter von zumindest neugeboren zumindest ein Organ einen xenogenen funktionellen Thymus und wahlweise xenogene Zellen, Gewebe und/oder Organe umfaßt, wobei die Zellen zumindest eine Art ausgewählt aus blutbildenden Stammzellen, unreifen, differenzierten, blutbildenden Zellen und reifen, differenzierten, blutbildenden Zellen umfassen.

4. Chimärer, tierischer Wirt nach Anspruch 3, worin das zumindest eine Organ in der Lage ist, vom Zeitpunkt des Einbringens in den Wirt weg zumindest drei Monate lang zu funktionieren.

5. Chimärer, tierischer Wirt nach Anspruch 3, worin das Tier ein Säugetier ist.

6. Chimärer, nicht-menschlicher, Säugetierwirt, umfassend:

einen immunodefizienten Säugetierwirt, dem funktionelle syngene Lymphozyten fehlen, in dem aber der Thymus vorhanden ist; und als Ergebnis des Einbringens der xenogenen Zellen und des Organs oder dessen Vorläufers in den tierischen Wirt mit einem Alter von zumindest neugeboren

xenogene Zellen und zumindest ein Organ oder einen Vorläufer davon, worin das zumindest eine Organ einen funktionellen Thymus und die Zellen zumindest eine Art ausgewählt aus blutbildenden Stammzellen, unreifen, differenzierten, blutbildenden Zellen und reifen, differenzierten, blutbildenden Zellen umfassen.

7. Chimärer Säugetierwirt nach Anspruch 6, worin das zumindest eine Organ weiters zumindest eine Art ausgewählt aus Lymphknoten, Lebergewebe und genetischen Vorläufern davon umfaßt.

8. Chimärer Säugetierwirt nach Anspruch 6, worin die xenogenen Zellen menschliche Zellen sind und das Organ aus menschlichen Zellen besteht.

9. Chimärer Säugetierwirt nach Anspruch 8, worin der Säugetierwirt als Ergebnis von in vivo Reifung der Stammzellen, die in den Säugetierwirt eingebracht wurden, reife, differenzierte, menschliche, blutbildende Zellen umfaßt.

10. Chimärer Säugetierwirt nach Anspruch 9, worin die Stammzellen von einem menschlichen Wirt mit einem Alter von zumindest neugeboren stammen.

11. Chimärer Säugetierwirt nach Anspruch 6, worin die xenogenen Zellen und das Organ beim Einbringen in den Säugetierwirt menschlich und fötal sind.

12. Chimärer Säugetierwirt nach Anspruch 11, worin der Säugetierwirt als Ergebnis von in vivo Reifung der Stammzellen, die in den Säugetierwirt eingebracht wurden, menschliche, reife, periphere Blutlymphozyten umfaßt.

13. Chimärer Säugetierwirt nach Anspruch 12, worin die xenogenen Zellen oder das Organ mit einem Pathogen infiziert werden.

14. Chimärer, streng kombinierter, immunodefizienter Mäusewirt, umfassend:

xenogene Zellen und zumindest ein Organ oder Vorläufer davon, worin das zumindest eine Organ einen funktionellen Thymus und die Zellen als Ergebnis des Einbringens der xenogenen Zellen und des Organs oder dessen Vorläufers in den Mäusewirt mit einem Alter von zumindest neugeboren zumindest eine Art ausgewählt aus blutbildenden Stammzellen, unreifen, differenzierten, blutbildenden Zellen und reifen, differenzierten, blutbildenden Zellen umfassen.

15. Mäusewirt nach Anspruch 14, worin das zumindest eine Organ weiters zumindest eine Art ausgewählt aus Lymphknoten, Lebergewebe und Vorläufern davon umfaßt.

16. Mäusewirt nach Anspruch 14, worin die xenogenen Zellen menschliche Zellen sind und das Organ aus menschlichen Zellen besteht.

**17.** Mäusewirt nach Anspruch 16, worin der Mäusewirt als Ergebnis von in vivo Reifung der Stammzellen, die in den Mäusewirt eingebracht wurden, reife, differenzierte, blutbildende Zellen umfaßt.

**18.** Mäusewirt nach Anspruch 17, worin die differenzierten, blutbildenden Zellen periphere Blutlymphozyten umfassen.

**19.** Mäusewirt nach Anspruch 14, worin die Stammzellen aus einer Säugetierquelle mit einem Alter von zumindest neugeboren stammen.

**20.** Mäusewirt nach Anspruch 14, worin die xenogenen Zellen und das Organ beim Einbringen in den Mäusewirt fötal sind.

**21.** Mäusewirt nach Anspruch 20, worin der Mäusewirt als Ergebnis von in vivo Reifung der Stammzellen, die in den Mäusewirt eingebracht wurden, menschliche, reife, differenzierte, blutbildende Zellen umfaßt.

**22.** Mäusewirt nach Anspruch 21, worin die xenogenen Zellen oder das Organ mit einem Pathogen infiziert werden.

**23.** Mäusewirt nach Anspruch 22, worin das Pathogen HIV ist.

**24.** Mäusewirt nach Anspruch 21, worin das zumindest eine Organ weiters zumindest einen Lymphknoten oder einen Vorläufer davon und der Mäusewirt weiters menschliche Antikörper umfaßt.

**25.** Verfahren zur Herstellung von menschlichen Plasmazellen, wobei das Verfahren umfaßt:
das Züchten eines chimären, streng kombinierten, immunodefizienten Mäusewirts, umfassend als Ergebnis des Einbringens der xenogenen Zellen und des Organs oder des Vorläufers davon in den Mäusewirt mit einem Alter von zumindest neugeboren:
menschliche Zellen, zumindest zwei Organe, worin die zumindest zwei Organe einen funktionellen Thymus und einen funktionellen Lymphknoten und die Zellen zumindest eine Art ausgewählt aus blutbildenden Stammzellen, unreifen, differenzierten, blutbildenden Zellen oder reifen, differenzierten, blutbildenden Zellen, umfassen, und ein von den reifen, differenzierten, blutbildenden Zellen erkanntes Antigen, wodurch Plasmazellen produziert werden.

**26.** Verfahren zur Herstellung von menschlichem Immunoglobulin, wobei das Verfahren umfaßt:
das Isolieren von Plasmazellen oder Vorläufern davon aus einem chimären, streng kombinierten, immunodefizienten Mäusewirt, umfassend als Ergebnis des Einbringens der xenogenen Zellen und des Organs oder des Vorläufers davon in den Mäusewirt mit einem Alter von zumindest neugeboren:
xenogene Zellen, zumindest ein Organ, worin das zumindest eine Organ einen funktionellen Thymus und die Zellen reife, differenzierte, blutbildende Zellen, umfassen, und Plasmazellen als Ergebnis der Gegenwart eines von den reifen, differenzierten, blutbildenden Zellen erkannten Antigens;
das Immortalisieren der isolierten Plasmazellen oder der Vorläufer davon, um eine immortalisierte Zelle bereitzustellen, die Immunoglobulin sekretiert; und
das Screenen der immortalisierten Zellen auf die Produktion von Immunoglobulin.

**27.** Verfahren zum Bestimmen der Wirkung einer Variation einer Bedingung auf Krankheitssymptome oder durch einen Krankheitszustand hervorgerufenen Indikationen in einem Säugetierwirt, wobei das Verfahren umfaßt:
das Hervorrufen eines Krankheitszustands in einem chimären, streng kombinierten, immunodefizienten Mäusewirt, umfassend als Ergebnis des Einbringens der xenogenen Zellen und des Organs oder des Vorläufers davon in den Mäusewirt mit einem Alter von zumindest neugeboren:
xenogene Zellen, zumindest ein Organ, worin das zumindest eine Organ einen funktionellen Thymus und die Zellen zumindest eine Art ausgewählt aus blutbildenden Stammzellen, unreifen, differenzierten, blutbildenden Zellen und reifen, differenzierten, blutbildenden Zellen, umfassen, und ein zweites Organ, falls das Krankheitssymptom oder die Indikation mit einem derartigen zweiten Organ assoziiert ist;
das Unterwerfen des Mäusewirts der Variation; und
das Bestimmen der Auswirkung der Variation auf das Krankheitssymptom oder die Indikation.

**28.** Verfahren nach Anspruch 27, worin die Variation die Verabreichung eines Arzneimittels ist.

**29.** Verfahren nach Anspruch 27, worin die Variation die Verabreichung von für den Krankheitszustand spezifischen, lymphozytischen oder myelomonozytischen Zellen ist.

**30.** Verfahren zur Herstellung eines chimären Wirts, der zur Verarbeitung von xenogenen Stammzellen befähigt ist, wobei das Verfahren umfaßt:

das Einbringen an einer Stelle, wo sich xenogene Zellen vermehren können, von fötalem Thymusgewebe, und von xenogenen Stammzellen an derselben oder einer davon verschiedenen Stelle, in eine streng kombinierte, immunodefiziente Maus.

**31.** Verfahren nach Anspruch 30, umfassend den zusätzlichen Schritt des Einbringens zumindest einer Art ausgewählt aus xenogenem Lymphknotengewebe und Lebergewebe.

**32.** Verfahren nach Anspruch 31, worin die xenogenen Zellen und das Gewebe menschliche Zellen und Gewebe sind.

**33.** Verfahren nach Anspruch 31, worin das Alter des fötalen Thymusgewebes etwa 9 - 24 Schwangerschaftswochen und das des Lymphknotengewebes mehr als etwa 15 Schwangerschaftswochen beträgt.

**34.** Verfahren nach Anspruch 33, worin die xenogenen Zellen und das Gewebe menschliche Zellen und Gewebe sind.

**35.** Verfahren nach Anspruch 30, worin die Stelle für das Thymusgewebe die Nierenkapsel ist.

**36.** Verfahren nach Anspruch 30, worin der Mäusewirt in einer anderen physiologischen Funktion als der des blutbildenden Systems einen Defekt aufweist.

**37.** Verfahren zum Bestimmen der Gegenwart von Stammzellen in einer ein Zellgemisch umfassenden Zusammensetzung, wobei das Verfahren umfaßt:

das Aufteilen der Zellen in Fraktionen auf der Basis von Oberflächenmembran-Proteinmarkern;

das Einbringen von zumindest einer der Zellfraktionen in eine Maus nach Anspruch 14;

das Reifen-Lassen der Stammzellen in dem Mäusewirt;

das Entfernen eines Ursprungs von peripheren Blutlymphozyten aus dem Mäusewirt; und

das Bestimmen der Gegenwart von differenzierten Zellen aus den Stammzellen als Nachweis für die Stammzellen in der Fraktion.

**38.** Verfahren zur Herstellung einer Teilmenge von menschlichen, blutbildenden Zellen, wobei das Verfahren umfaßt:

das Einbringen von menschlichen, blutbildenden Stammzellen in einen Mäusewirt nach Anspruch 14, zusammen mit zumindest einem Lymphokin zum Bewirken von Differenzierung in zumindest eine der blutbildenden Linien;

das Halten der Wirte über eine für die Stammzellen ausreichende Zeit zur Differenzierung und Fortpflanzung, um Zellen der Teilmenge zu produzieren; und

das Ernten der Teilmenge von menschlichen, blutbildenden Zellen.

**39.** Verfahren zur Herstellung von modifizierten, menschlichen Zellen, umfassend das Aussetzen von menschlichen Zellen der Umgebung eines chimären, immunodefizienten, nicht-menschlichen, tierischen Wirts, der zumindest einen Abschnitt seines Immunsystems, dem syngene Lymphozyten fehlen, umfaßt, in dem jedoch der Thymus vorhanden ist, ohne Zellen des Wirtsäugetiers der Modifikation zu unterziehen.

**40.** Chimäres, immunodefizientes, nicht-menschliches Säugetier, das menschliche Zellen enthält, wobei die menschlichen Zellen als Ergebnis der Umgebung in dem Säugetier spezifisch modifiziert sind, wobei das Säugetier zumindest einen Teil seines Immunsystems besitzt, ihm syngene Lymphozyten fehlen, in dem jedoch der Thymus vorhanden ist.

**41.** Verfahren oder Säugetier nach Anspruch 39 oder 40, worin die Zellen als Teil eines Organs vorliegen.

**42.** Verfahren oder Säugetier nach Anspruch 39, 40 oder 41, worin die menschlichen Zellen Lymphozytenzellen sind und die Modifikation in den Zellen ein Immunrespons ist.

**43.** Verfahren oder Säugetier nach Anspruch 39, 40 oder 41, worin die menschlichen Zellen Ziel eines Injektionswirkstoffes sind und die Modifikation eine Injektion der Zellen ist.

**44.** Verfahren oder Säugetier nach Anspruch 43, das zusätzlich einen therapeutischen Wirkstoff umfaßt, der mit den injizierten Zellen in Kontakt tritt.

**45.** Säugetier nach irgendeinem der Ansprüche 40 - 44, worin die menschlichen Zellen aus dem Lymphknoten stammen oder der Lymphknoten sind und das Säugetier zur Verwendung beim Testen auf HIV dient.

**Revendications**

**1.** Hôte animal non humain, chimérique, comprenant :
un hôte animal immunodéficient comprenant au moins une partie d'un système immunitaire, qui n'a pas de lymphocytes syngéniques fonctionnels, mais dans lequel le thymus est présent; et
du matériel cellulaire xénogénique, qui est un organe ou un tissu xénogénique, capable de fonctionner dans l'hôte, ou qui comprend des cellules xénogéniques conjointement avec un tel organe ou tissu, si cela est nécessaire pour la maturation des cellules, le matériel cellulaire xénogénique étant introduit dans l'hôte animal à un âge d'au moins nouveau-né.

**2.** Hôte animal chimérique selon la revendication 1, dans lequel ledit hôte animal est immunodéficient en raison d'une déficience génétique des cellules lymphoïdes.

**3.** Hôte animal non humain, chimérique, comprenant :
un hôte animal immunodéficient comprenant au moins une partie d'un système immunitaire, qui n'a pas de lymphocytes syngéniques fonctionnels, mais dans lequel le thymus est présent; et
dans lequel au moins un organe comprend un thymus xénogénique fonctionnel et optionnellement, des cellules, tissus et/ou organes xénogéniques, lesdites cellules comprenant au moins un type parmi les cellules souches hématopoïétiques, les cellules hématopoïétiques différenciées immatures ou les cellules hématopoïétiques différenciées matures, comme résultat de l'introduction desdits cellules, tissu et/ou organe xénogéniques dans ledit hôte animal à un âge d'au moins nouveau-né.

**4.** Hôte animal chimérique selon la revendication 3, dans lequel au moins un organe est capable de fonctionner pendant au moins trois mois à partir du moment de l'introduction dans ledit hôte.

**5.** Hôte animal chimérique selon la revendication 3, où ledit animal est un mammifère.

**6.** Hôte mammifère non humain chimérique comprenant :
un hôte mammifère immunodéficient, qui n'a pas de lymphocytes syngéniques fonctionnels, mais dans lequel le thymus est présent; et
des cellules et au moins un organe xénogéniques ou un progéniteur de ceux-ci, ledit au moins un organe comprenant un thymus fonctionnel et lesdites cellules comprenant au moins un type parmi les cellules souches hématopoïétiques, les cellules hématopoïétiques différenciées immatures ou les cellules hématopoïétiques différenciées matures, comme résultat de l'introduction desdits cellules et organe xénogéniques ou du progéniteur de ceux-ci dans ledit hôte animal à un âge d'au moins nouveau-né.

**7.** Hôte mammifère chimérique selon la revendication 6, dans lequel ledit au moins un organe comprend en outre au moins l'un des ganglions lymphatiques, du tissu hépatique ou d'un progéniteur de ceux-ci.

**8.** Hôte mammifère chimérique selon la revendication 6, dans lequel lesdites cellules xénogéniques sont des cellules humaines et ledit organe est constitué de cellules humaines.

**9.** Hôte mammifère chimérique selon la revendication 8, dans lequel ledit hôte mammifère comprend des cellules hématopoïétiques humaines différenciées matures, comme résultat de la maturation in vivo desdites cellules souches introduites dans ledit hôte mammifère.

10. Hôte mammifère chimérique selon la revendication 9, dans lequel lesdites cellules souches proviennent d'un hôte humain à l'âge d'au moins nouveau né.

11. Hôte mammifère chimérique selon la revendication 6, dans lequel lesdits cellules et organe xénogéniques sont humains et foetaux, lorsqu'ils sont introduits dans ledit hôte mammifère.

12. Hôte mammifère chimérique selon la revendication 11, dans lequel ledit hôte mammifère comprend des lymphocytes humains matures du sang périphérique, comme résultat de la maturation in vivo desdites cellules souches introduites dans ledit hôte mammifère.

13. Hôte mammifère chimérique selon la revendication 12, dans lequel lesdits cellules ou organe xénogéniques sont infectés par un agent pathogène.

14. Souris hôte chimérique présentant une immunodéficience combinée sévère, comprenant :
des cellules et au moins un organe xénogéniques ou un progéniteur de ceux-ci, ledit au moins un organe comprenant un thymus fonctionnel et lesdites cellules comprenant au moins un type parmi les cellules souches hématopoïétiques, les cellules hématopoïétiques différenciées immatures ou les cellules hématopoïétiques différenciées matures, comme résultat de l'introduction desdits cellules et organe xénogéniques ou du progéniteur de ceux-ci dans ladite souris hôte à un âge d'au moins nouveau-né.

15. Souris hôte selon la revendication 14, dans laquelle ledit au moins un organe comprend en outre au moins l'un des ganglions lymphatiques, du tissu hépatique ou d'un progéniteur de ceux-ci.

16. Souris hôte selon la revendication 14, dans laquelle lesdites cellules xénogéniques sont humaines et ledit organe est constitué de cellules humaines.

17. Souris hôte selon la revendication 16, ladite souris comprenant des cellules hématopoïétiques différenciées matures comme résultat de la maturation in vivo desdites cellules souches introduites dans ladite souris hôte.

18. Souris hôte selon a revendication 17, dans laquelle lesdites cellules hématopoïétiques différenciées comprennent des lymphocytes du sang périphérique.

19. Souris hôte selon la revendication 14, dans laquelle les cellules souches proviennent d'un mammifère d'un âge d'au moins nouveau-né.

20. Souris hôte selon la revendication 14, dans laquelle lesdits cellules et organe xénogéniques sont foetaux lorsqu'ils sont introduits dans ladite souris hôte.

21. Souris hôte selon la revendication 20, ladite souris hôte comprenant des cellules hématopoïétiques humaines matures différenciées, comme résultat de la maturation in vivo desdites cellules souches introduites dans ladite souris hôte.

22. Souris hôte selon la revendication 21, dans laquelle lesdits cellules ou organe xénogéniques sont infectés par un agent pathogène.

23. Souris hôte selon la revendication 22, dans laquelle l'agent pathogène est le HIV.

24. Souris hôte selon la revendication 21, dans laquelle ledit au moins un organe comprend au moins un ganglion lymphatique ou le progéniteur de celui-ci, et ladite souris hôte comprend en outre des anticorps humains.

25. Méthode de production de cellules de plasma humain, ladite méthode consistant à :
assurer la croissance d'une souris hôte chimérique présentant une immunodéficience combinée sévère, comprenant :

des cellules humaines, au moins deux organes, lesdits au moins deux organes comprenant un thymus fonctionnel et un ganglion lymphatique fonctionnel, et lesdites cellules comprenant au moins un type parmi les cellules souches hématopoïétiques, les cellules hématopoïétiques différenciées immatures ou les cellules hématopoïétiques différenciées matures, comme résultat de l'introduction desdits cellules et organe ou du progéniteur de ceux-ci dans ladite souris hôte à un âge d'au moins nouveau-né, et un antigène reconnu par lesdites cellules hématopoïétiques différenciées matures, des cellules plasmatiques étant ainsi produites.

26. Méthode de production d'immunoglobuline humaine, ladite méthode consistant à :

isoler des cellules de plasma ou un progéniteur de celles-ci à partir d'une souris hôte chimérique présentant une immunodéficience combinée sévère, comprenant:

des cellules xénogéniques, au moins un organe, ledit au moins un organe comprenant un thymus fonctionnel, et lesdites cellules comprenant des cellules hématopoïétiques différenciées matures, comme résultat de l'introduction desdits cellules et organe xénogéniques ou du progéniteur de ceux-ci dans ladite souris hôte à un âge d'au moins nouveau-né, et des cellules plasmatiques comme résultat de la présence d'un antigène reconnu par lesdites cellules hématopoïétiques différenciées matures;

immortaliser lesdites cellules plasmatiques isolées ou le progéniteur de celles-ci pour fournir une cellule immortalisée qui sécrète de l'immunoglobuline; et

sélectionner lesdites cellules immortalisées pour la production de l'immunoglobuline.

27. Méthode pour déterminer l'effet de la variation d'une condition sur les symptômes d'une maladie, ou les signes provoqués par un état maladif, chez un hôte mammifère, ladite méthode consistant à :

induire un état maladif dans une souris hôte chimérique présentant une immunodéficience combinée sévère, comprenant :

des cellules xénogéniques, au moins un organe, ledit au moins un organe comprenant un thymus fonctionnel et lesdites cellules comprenant au moins un type parmi les cellules souches hématopoïétiques, les cellules hématopoïétiques différenciées immatures ou les cellules hématopoïétiques différenciées matures, comme résultat de l'introduction desdits cellules et organe xénogéniques ou du progéniteur de ceux-ci dans ladite souris hôte à un âge d'au moins nouveau-né, et un second organe, lorsque ledit symptôme ou signe de la maladie est associé avec un tel second organe;

soumettre ladite souris hôte à ladite variation; et

déterminer l'effet de ladite variation sur ledit symptôme ou signe de la maladie.

28. Méthode selon la revendication 27, dans laquelle ladite variation est l'administration d'un médicament.

29. Méthode selon la revendication 27, dans laquelle ladite variation est l'administration de cellules lymphocytaires ou myélomonocytaires spécifiques dudit état maladif.

30. Méthode de production d'un hôte chimérique capable de transformer des cellules souches xénogéniques, ladite méthode consistant à :

introduire dans une souris présentant une immunodéficience combinée sévère, dans un site où les cellules xénogéniques peuvent croître, un tissu de thymus foetal et dans le même site ou dans un site différent, des cellules souches xénogéniques.

31. Méthode selon la revendication 30, comprenant l'étape additionnelle consistant à introduire au moins un parmi les tissus xénogéniques de ganglion lymphatique ou hépatique.

32. Méthode selon la revendication 31, dans laquelle lesdits cellules et tissu xénogéniques sont des cellules et tissu humains.

33. Méthode selon la revendication 31, dans laquelle ledit tissu de thymus foetal est d'un âge d'environ 9 à 24 semaines de gestation, et ledit tissu de ganglion lymphatique est d'un âge de plus d'environ 15 semaines de gestation.

34. Méthode selon la revendication 33, dans laquelle lesdits cellules et tissu xénogéniques sont des cellules et tissu humains.

**35.** Méthode selon la revendication 30, dans laquelle ledit site pour l'introduction dudit tissu de thymus est la capsule rénale.

**36.** Méthode selon la revendication 30, dans laquelle ladite souris hôte possède une fonction physiologique déficiente autre que le système hématopoïétique.

**37.** Méthode pour détecter la présence de cellules souches dans une composition comprenant un mélange de cellules, ladite méthode consistant à :

diviser des cellules en fractions en se basant sur des marqueurs protéiques de surface membranaire;

introduire au moins une desdites fractions cellulaires dans une souris hôte selon la revendication 14;

laisser lesdites cellules souches maturer dans lesdites souris hôtes;

enlever une source de lymphocytes de la circulation périphérique de ladite souris hôte; et

déterminer la présence de cellules différenciées à partir desdites cellules souches, indicatrice de la présence desdites cellules souches dans ladite fraction.

**38.** Méthode de production d'une sous-population de cellules hématopoïétiques humaines, ladite méthode consistant à:

introduire des cellules souches hématopoïétiques humaines dans une souris hôte selon la revendication 14, avec au moins une lymphokine pour provoquer la différenciation en au moins une des lignées hématopoïétiques;

maintenir lesdits hôtes pendant un temps suffisamment long pour que lesdites cellules souches se différencient et prolifèrent pour produire des cellules de ladite sous-population; et

récolter ladite sous-population de cellules hématopoïétiques humaines.

**39.** Méthode de production de cellules humaines modifiées consistant à soumettre des cellules humaines à l'environnement d'un hôte animal non humain immunodéficient chimérique comprenant au moins une partie de son système immunitaire, n'ayant pas de lymphocytes syngéniques, mais dans lequel le thymus est présent, sans soumettre les cellules de l'hôte mammifère à ladite modification.

**40.** Mammifère non humain immunodéficient chimérique comprenant des cellules humaines, les cellules humaines étant spécifiquement modifiées, par l'effet de l'environnement dans le mammifère, le mammifère ayant au moins une partie de son système immunitaire, n'ayant pas de lymphocytes syngéniques, mais ayant un thymus.

**41.** Méthode ou mammifère selon la revendication 39 ou 40, où les cellules sont présentes en tant que partie d'un organe.

**42.** Méthode ou mammifère selon la revendication 39, 40 ou 41, où les cellules humaines sont des cellules lymphocytaires et la modification dans les cellules est une réponse immunitaire.

**43.** Méthode ou mammifère selon la revendication 39, 40 ou 41 où les cellules humaines sont une cible pour un agent d'injection et la modification est une injection de cellules.

**44.** Méthode ou mammifère selon la revendication 43 comprenant, en outre, un agent thérapeutique qui entre en contact avec les cellules injectées.

**45.** Mammifère selon l'une quelconque des revendications 40 à 44, où les cellules humaines proviennent des ganglions lymphatiques ou sont les ganglions lymphatiques et le mammifère est destiné à être utilisé pour rechercher la présence de HIV.